(19) Europäisches Patentamt
European Patent Office
Office européen des brevets

(11) Publication number: 0 315 249 A1

# (12) EUROPEAN PATENT APPLICATION

(21) Application number: 88202371.6

(22) Date of filing: 25.10.88

(51) Int. Cl.4: A61K 31/50 , A61K 9/20

Claims for the following Contracting States: ES + GR.

(30) Priority: 02.11.87 US 115810

(43) Date of publication of application: 10.05.89 Bulletin 89/19

(84) Designated Contracting States: AT BE CH DE ES FR GB GR IT LI LU NL SE

(71) Applicant: MERCK & CO. INC.
126, East Lincoln Avenue P.O. Box 2000
Rahway New Jersey 07065-0900(US)

(72) Inventor: Bavitz, Joseph F.
2345 Terwood Road
Huntingdon Valley Pennsylvania 19006(US)
Inventor: Katdare, Ashok V.
215 W. Freedley Street
Norristown Pennsylvania 19401(US)

(74) Representative: Hesketh, Alan, Dr.
European Patent Department Merck & Co., Inc. Terlings Park Eastwick Road
Harlow Essex, CM20 2QR(GB)

## (54) Phthalazineacetic acid composition and tablet.

(57) A novel tablet containing a phthalazineacetic acid and a process for producing it is described.

EP 0 315 249 A1

# PHTHALAZINEACETIC ACID COMPOSITION AND TABLET

This invention is directed to a pharmaceutical tablet and a composition therefor.

## BACKGROUND OF THE INVENTION

The enzyme aldose reductase acts to catalytically convert aldoses such as glucose and galactose to their corresponding alditols. The alditols, thus formed, tend to accumulate in the cell giving rise to osmotic pressures which may impair the function of the cells. The enzyme acts primarily when the concentration of aldose is high such as in diabetics, thus giving rise to clinical conditions in diabetes such as retinopathy, neuropathy, nephropathy, and the like. Certain phthalazin-4-ylacetic acid compounds have been found to be useful in the reduction or prevention of the clinical effects associated with diabetes. These compounds are described in U.S. Patents 4,251,528 and 4,393,062. A particularly useful compound is 2-(2-fluoro-4-bromo benzyl)-1,2-dihydro-1-oxophthalazin-4-ylacetic acid which may be identified also by the Chemical Abstracts system of nomenclature as 3-((4-bromo-2-fluorophenyl)methyl)-3,4 dihydro-4-oxo-1-phthalazineacetic acid and represented by Formula I.

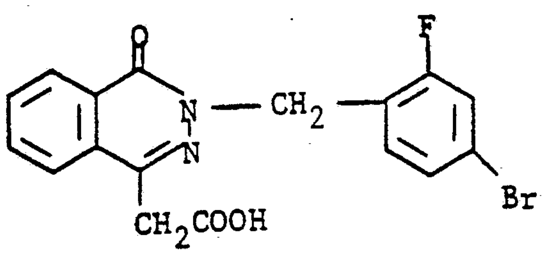

(I)

A pharmaceutical tablet of the compound of Formula I has been disclosed in the aforecited U.S. 4,393,062. However, it has been found that when tablets are prepared with the formulation as described in said patent or are prepared using a number of the conventional tablet formulating ingredients, the amount and/or kind of tablet ingredients are such as to necessitate for the required dose of drug, either a large tablet or a tablet containing less than the quantity of drug desired for said dose, thereby requiring multiple tablets. It is desirable to have a smaller tablet containing the required dosage which should be easier to swallow encouraging patient compliance which tablet also has the appropriate physicochemical properties for efficient and effective utilization of the drug such as rapid disintegration and dissolution.

## STATEMENT OF INVENTION

The present invention is directed to an improved pharmaceutical tablet comprising the compound of Formula I disclosed and claimed in the aforecited U.S. 4,251,528 and 4,393,062 and hereinafter referred to as the phthalazineacetic acid compound. The tablets according to the present invention have superior physico-chemical properties and are of a size allowing for single rather than multiple dosage administration.

## DESCRIPTION OF THE INVENTION

The present invention is directed to a new composition which comprises a blend of the phthalazineacetic acid compound and certain inert tablet ingredients with a minimal amount of processing aids and to the tablets formed therewith.

More specifically, the composition includes the phthalazineacetic acid compound, 3-((4 bromo-2-fluorophenyl)methyl-3,4-dihydro-4-oxo-1-phthalazineacetic acid.

The tablet composition of the present invention contains by weight from about 83 to 88 percent of the phthalazineacetic acid compound, about 8 to 20 percent of a diluent, about 1 to 4 percent of a disintegrant,

about 1 to 4 percent of a binder and about 0.5 to 2 percent of a lubricant.

A suitable diluent is microcrystalline cellulose. Suitable disintegrants include various modified starches and modified cellulose polymers, preferably croscarmellose sodium. Suitable binders include gelatin, modified starches, preferably pregelatinized starch. Suitable lubricants include magnesium or calcium stearate, preferably magnesium stearate. It is desirable to include a small amount (about 0.25 to 0.35 percent) of wetting agent such as sodium lauryl sulfate or docusate sodium (dioctyl sulfosuccinate sodium).

The preferred tablet composition of the present invention contains by weight from about 83 to 88 percent 3-((4-bromo 2-fluorophenyl)methyl)3,4-dihydro-4-oxo-1-phthalazinoacetic acid, about 8 to 20 percent microcrystalline cellulose, about 1 to 4 percent pregelatinized starch, about 1 to 4 percent croscarmellose sodium, and about 0.5 to 1.5 percent magnesium stearate, and about 0.25 to 0.35 percent sodium lauryl sulfate.

Representative of the most preferred tablets are 300 mg and 600 mg tablets of the following compositions.

TABLE I

| Component | TABLET A | TABLET B |
|---|---|---|
| | mg/tablet | mg/tablet |
| Compound Formula I | 300.0 | 600.0 |
| Pregelatinized starch (NF 1551) | 10.0 | 20.0 |
| Sodium lauryl sulfate NF | 1.0 | 2.0 |
| Microcrystalline cellulose NF | 31.0 | 62.0 |
| Croscarmellose Sodium NF | 5.0 | 10.0 |
| Magnesium stearate NF | 3.0 | 6.0 |

The tablets formed by the foregoing composition are consistent in quality, hold the required relatively high doses in a size suitable for gaining patient compliance and generally have excellent physico-chemical qualities. Thus, for example, the foregoing 300 mg tablet has been found to have the following properties:

| | |
|---|---|
| Tablet weight | 350 milligrams |
| Tablet hardness[1] | 12-14 kps. |
| Disintegration time[2] | 1 to 2 minutes |
| Friability[3] | 0.16 percent |
| Dissolution[4] 10 minutes | 77% |
| Dissolution 20 minutes | 88% |
| Dissolution 30 minutes | 90% |

[1] Schleuniger hardness tester
[2] USP disintegration apparatus
[3] Roche friability tester, 16 minutes
[4] USP dissolution method, apparatus 2

Tablets having higher and lower doses of the drug have similar desirable properties when produced in the

above described composition range.

The tablets may be prepared by (1) mixing the phthalalazineacetic acid compound. pregelatinized starch and microcrystalline cellulose in a planetary mixer or a high speed mixer for time sufficient to insure thorough mixing; (2) dissolving sodium lauryl sulfate in a suitable amount of water at room temperature; (3) adding aqueous mixture of Step (2) to the powder mixture of Step (1) to granulate the powder mixture; (4) passing the granulated mixture through a screen having an opening of 1.7 to 2.4 mm; (5) drying the sized granulated mixture at about 40 60° C, preferably 50° C for time sufficient to effect drying; (6) passing the dried granulated mixture through a screen having an opening of 0.55 to 0.65 mm; (7) mixing the dry-sized granulation with croscarmellose sodium; (8) adding magnesium stearate which has previously been bolted through a No. 60 screen (0.25 mm opening); (9) blending the resulting mix for time sufficient to obtain the desired lubricated granular tablet formulation, usually about 2 to 5 minutes.

Thereafter, the lubricated granular formulation is compressed using a conventional tablet press to form tablets which are thereafter preferably coated. Substances which may be used for coating include hydroxypropylmethylcellulose, hydroxypropylcellulose, titanium oxide, talc and colorants.

The following examples illustrate the invention but are not to be construed as limiting.

## EXAMPLE I

300.0 grams of 3-((4-bromo-2-fluorophenyl) methyl)-3,4-dihydro-4-oxo-1-phthalazine-acetic acid (previously milled in an Alpine Mill), 16.5 grams of pregelatinized starch NF 1551, and 72.0 grams microcrystalline cellulose NF (Avicel PH 101) were mixed together in a planetary mixer for 5 minutes. The resulting mixture was passed through a No. 30 sieve (0.59 mm sieve opening). The screened material was remixed for 5 additional minutes.

One gram of sodium lauryl sulfate was dissolved in 110 milliliters of water and the resulting solution was added to the above powdery mixture over a period of one minute at 45 to 50 rpm, then for a total of 5 minutes at 60 to 70 rpm. The resulting granulate was passed through a No. 10 screen (2.00 mm sieve opening). The screened granulate was then dried at 50° C in a fluid bed dryer for approximately 15 minutes. The dried material was sized by passing through sieve No. 20 (0.84 mm sieve opening). 8.0 grams of croscarmellose sodium NF was added, and the resulting mixture blended for 5 minutes in a V-shaped blender.

4.0 grams of magnesium stearate NF was bolted through a No. 60 sieve and to it was added the dry-sized granulation and the resulting mixture blended for 3 minutes to obtain the desired lubricated granular tablet composition.

The composition was fed into a standard single punch tablet press to obtain 1000 tablets of about 9/32 x 16/32 inches in size.

## Claims

1. A tablet composition containing by weight from about 83 to 88 percent of a phthalazine acetic acid compound, about 8 to 20 percent diluent, about 1 to 4 percent binder, about 1 to 4 percent disintegrant, about 0.5 to 2 percent lubricant and 0.25 to 0.35 percent wetting agent.

2. A tablet composition containing by weight from about 83 to 88 percent of a phthalazineacetic acid compound, about 8 to 20 percent microcrystalline cellulose, about 1 to 4 percent pregelatinized starch, 1 to 4 percent croscarmellose sodium, 0.5 to 1.5 percent magnesium stearate and 0.25 to 0.35 percent sodium lauryl sulfate.

3. A composition of Claim 1 in which the phthalazineacetic acid compound is 3-((4-bromo-2-fluorophenyl)methyl)-3,4-dihydro-4-oxo-1-phthalazineacetic acid having the formula

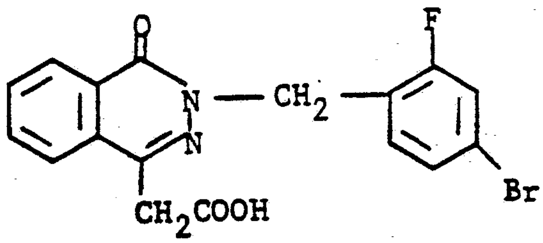

4. A tablet prepared from the composition of Claim 2.

5. A tablet according to Claim 4 which is film coated.

Claims for the following Contracting States: ES, GR

1.- A process for preparing a pharmaceutical composition containing a phthalazineacetic acid compound as active ingredient, to be orally administered, which comprises:

1) mixing:

- from about 83 to 88 percent by weight of the phthalazineacetic acid compound;
- from about 1 to 4 percent by weight of a suitable binder; and
- from about 8 to 20 percent by weight of a suitable diluent in a planetary mixer or a high speed mixer for suffient time to insure thorough mixing;

2) dissolving from about 0.25 to 0.35 percent by weight of a suitable wetting agent in a suitable amount of water at room temperature;

3) adding the aqueous mixture of step 2) to the powder mixture of step 1) to granulate the powder mixture;

4) passing the granulated mixture through a screen having an opening of 1.7 to 2.4 mm;

5) drying the sized granulated mixture at about 40 - 60 degrees C.

6) passing the dried granulated mixture through a screen having an opening of 0.55 to 0.65 mm;

7) mixing the dry-sized granulation with a suitable disintegrant in a range of from about 1 to 4 percent by weight thereof;

8) adding a suitable lubricant in a range from about 0.5 to 2% by weight thereof; and

9) blending the resulting mix for sufficient time to obtain the desired lubricated granular formulation.

2.- A process according to claim 1 wherein the composition obtained contains by weight from
- about 83 to 88 percent of phthalazineacetic compound;
- about 8 to 20 percent of diluent;
- about 1 to 4 percent of binder;
- about 1 to 4 percent of disintegrant;
- about 0.5 to 1.5 percent of lubricant and
- about 0.25 to 0.35 percent of wetting agent.

3.- A process according to claims 1 and 2, characterized in that:
- the suitable binder is pregelatinized starch;
- the suitable diluent is microcrystalline cellulose;
- the suitable wetting agent is sodium lauryl sulfate;
- the suitable disintegrant is croscarmellose sodium; and
- the suitable lubricant is magnesium stearate which has been previously bolted through a screen having an opening of 0.25 mm.

4.- A process according to claims 1 to 3, characterized in that the phthalazineacetic compound is 3- [(4-bromo-2-fluorophenyl)methyl]-3,4-dihydro-4-oxo-1-phthalazineacetic acid having the following formula

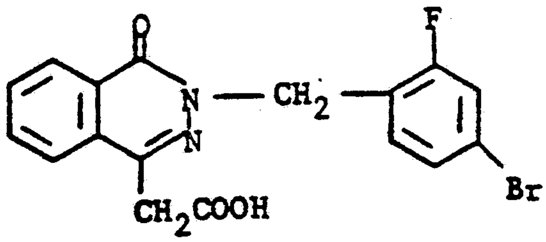

5.- A process for preparing a tablet containing the pharmaceutical composition obtained by claim1, which comprises

a) compressing said lubricated granular formulation using a conventional tablet press to form tablets, and

b) coating said tablets previously obtained with a suitable coating substance such as hydroxypropylmethylcellulose, hydroxypropylcellulose, titanium oxide, talc and colorants.

6.- A process according to claim 5 wherein the tablet prepared contains a composition containing by weight from:

- about 83 to 88 percent of a phthalazineacetic acid compound;
- about 8 to 20 percent of microcrystalline cellulose;
- about 1 to 4 percent of pregelatinized starch;
- about 1 to 4 percent of croscarmellose sodium;
- about 0.5 to 1.5 percent of magnesium stearate; and
- about 0.25 to 0.35 percent of sodium lauryl sulfate.

7.- A process according to claim 5 in which, in the tablet obtained, the phthalazineacetic acid compound is 3-[(4-bromo-2-fluorophenyl)methyl]-3,4-dihydro-4-oxo-phthalazineacetic acid having the following formula

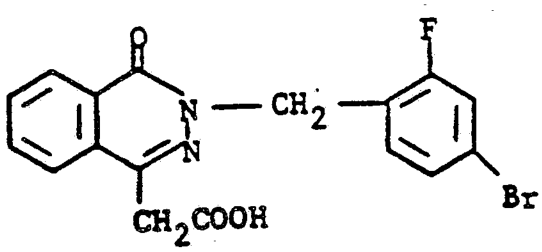

8.- A process according to claims 5 to 7 wherein the tablet obtained is film coated.

European Patent Office

# EUROPEAN SEARCH REPORT

Application Number

EP 88 20 2371

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 393 062 (D.R. BRITTAIN et al.)<br>* Example 59; claims 1-5 *<br>--- | 1-5 | A 61 K 31/50<br>A 61 K 9/20 |
| Y | EP-A-0 189 114 (MERCK & CO., INC.)<br>* Page 3, lines 1-25 *<br>--- | 1-5 | |
| Y | EP-A-0 130 683 (MALLINCKRODT, INC.)<br>* Page 8, line 8 - page 11, line 13; page 19, lines 1-30 *<br>--- | 1-5 | |
| Y | EP-A-0 237 241 (AMERICAN HOME PRODUCTS CORP.)<br>* Page 4, lines 35-48; claims 1-10 *<br>----- | 1-5 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 27-01-1989 | TZSCHOPPE,D.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document